# EUROPEAN PATENT APPLICATION

(11) **EP 4 517 406 A1**
(43) Date of publication of application: **05.03.2025**
(21) Application number: 23793987.1
(22) Date of filing: 14.04.2023
(51) Int. Cl.: G02B 21/32, C12M 1/00, G01N 37/00

(54) **MANIPULATION SYSTEM AND FLUID CHIP**

(30) Priority: 26.04.2022 JP 2022072418
(71) Applicant: University of Yamanashi, Kofu-shi, Yamanashi 400-8510 (JP)
(72) Inventor: UKITA, Yoshiaki, Kofu-shi, Yamanashi 400-8510 (JP); ABE, Takaaki, Kofu-shi, Yamanashi 400-8510 (JP)
(74) Representative: BSB Patentanwälte Schütte & Engelen Partnerschaft mbB
(86) International application number: PCT/JP2023/015165
(87) International publication number: WO 2023/210407

(57) **Abstract**

To provide a technique for facilitating position control of an object to be manipulated.

A manipulation system for manipulating disposition of an object to be manipulated at a predetermined position in a manipulation region containing liquid, the manipulation system including: the manipulation region; a first channel that is a plurality of channels connected to the manipulation region and containing the liquid; a liquid control unit configured to move the liquid in the first channel; and a second channel that is a series of channels containing the liquid, the second channel being connected to each of the plurality of first channels, is made.

## Description

### TECHNICAL FIELD

The present invention relates to a manipulation system and a fluid chip.

### BACKGROUND ART

In recent years, a technique called micro total analysis systems (MicroTAS) that executes a biochemical process using a micro channel or the like has been actively developed. MicroTAS is used, for example, for analysis of cell-cell interaction, construction of a system in which multiple cells are arranged in the field of regenerative medicine, and the like. There has been known a conventional technique for moving an in-liquid object to be manipulated to a target position by moving liquid in a channel containing the liquid (see, for example, Patent Literature 1).

### CITATIONS LIST

### PATENT LITERATURE

Patent Literature 1: JP 2021-185782 A

### SUMMARY OF INVENTION

### TECHNICAL PROBLEMS

It is typically difficult to control movement of an in-liquid object to be manipulated to a desired position by moving liquid. One of the reasons why the difficulty of control is high is that the object to be manipulated is indirectly moved using the liquid. Since liquid does not have a fixed shape, the behavior of the liquid and the object to be manipulated around the liquid cannot be easily predicted at the time of simple repetition of an operation of applying a predetermined pressure to the liquid, which makes it difficult to perform position control of the object to be manipulated.

The present invention has been made in view of the above problems, and an object of the present invention is to provide a technique for facilitating position control of an object to be manipulated.

### SOLUTIONS TO PROBLEMS

In order to achieve the above object, a manipulation system of the present invention includes: a manipulation region containing liquid and an object to be manipulated whose position in the liquid is manipulated; a first channel that is a plurality of channels connected to the manipulation region and containing the liquid; a liquid control unit configured to move the liquid in the first channel; and a second channel that is a series of channels containing the liquid, the second channel being connected to each of the plurality of first channels.

That is, the manipulation region contains the liquid and the object to be manipulated in the liquid, and the liquid in the first channel can be moved in the first channel connected to the manipulation region. The plurality of first channels are provided, and the liquid flows into the manipulation region from the first channels and flows out from the manipulation region to the first channels by the movement of the liquid in the first channels. Therefore, the liquid also moves in the manipulation region in conjunction with the movement of the liquid in the first channels, and as a result, the object to be manipulated moves in the manipulation region. In this configuration, when the liquid in the first channels moves, a pressure locally fluctuates, and eventually a steady state is restored in which the pressure is made uniform. If such a pressure fluctuation locally occurs or the pressure fluctuation remains for a long time, it becomes difficult to control repeated movement of the liquid in first fluids.

To address the problem, the second channel is provided in the manipulation system. The second channel is a series of channels containing the liquid, and is connected to each of the plurality of first channels. Therefore, the second channel forms a channel connecting the plurality of first channels to each other. As a result, the pressures in the plurality of first channels are made uniform, and a local pressure difference hardly occurs. In addition, even if the pressure fluctuates due to the movement of the liquid in a specific first channel, the pressure fluctuation is made uniform at an early stage. Therefore, in a case where the position of the object to be manipulated is manipulated by repeating the movement of the liquid, it is possible to facilitate position control of the object to be manipulated.

### BRIEF DESCRIPTION OF DRAWINGS

Fig. 1A is a diagram illustrating a schematic configuration of a manipulation system, and Fig. 1B is a diagram illustrating a main part of the manipulation system.
Fig. 2A is a diagram illustrating an example of an image captured by a microscope, Fig. 2B is a diagram illustrating the shape of a manipulation region, and Figs. 2C to 2E are diagrams illustrating the structure of a switching part.
Fig. 3 is a block diagram illustrating a configuration of the manipulation system.
Fig. 4A is a diagram illustrating a simulation model, and Fig. 4B is a diagram illustrating a machine learning model.
Fig. 5 is a diagram for explaining reinforcement learning.
Fig. 6 is a flowchart of a reinforcement learning process.
Fig. 7 is a flowchart of a manipulation process.
Figs. 8A to 8C are diagrams illustrating an example of moving an object to be manipulated to a target position.
Fig. 9 is a diagram illustrating an example of a configuration for introducing an object to be manipulated into the manipulation region.
Fig. 10 is a diagram illustrating a substrate for realizing a manipulation system including a plurality of manipulation regions.
Fig. 11 is an enlarged view of the substrate for realizing the manipulation system including a plurality of manipulation regions.

### DESCRIPTION OF EMBODIMENTS

Here, an embodiment of the present invention will be described in the following order.
(1) Configuration of Manipulation System:
(2) Machine Learning Process Using Artificial Intelligence (AI):
(3) Manipulation Process:
(4) Other Embodiments:

### (1) Configuration of Manipulation System:

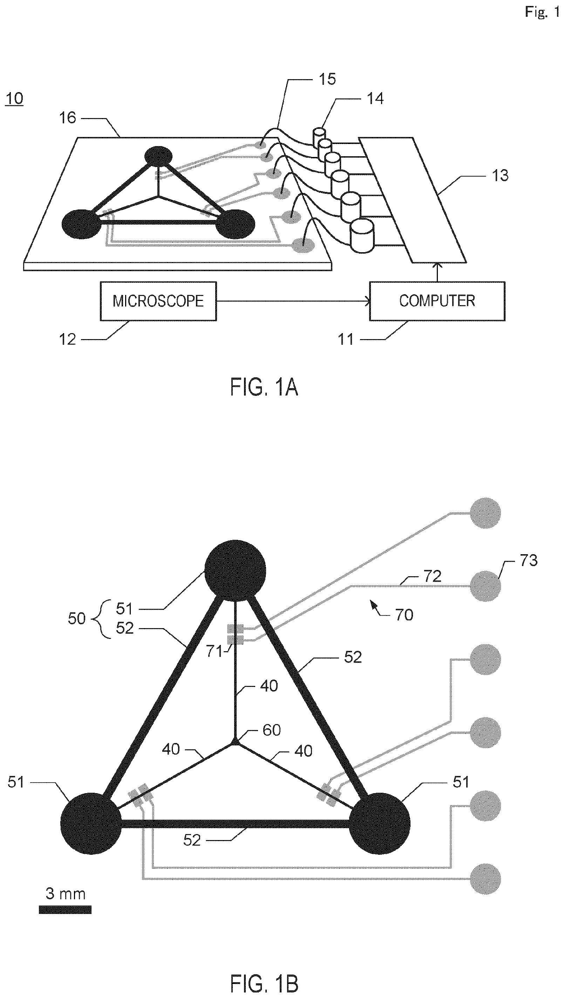

Fig. 1A is a diagram illustrating a schematic configuration of a manipulation system, and Fig. 1B is a diagram illustrating a main part of a manipulation system 10. The manipulation system 10 according to the present embodiment includes a computer 11, a microscope 12, a signal conversion interface 13, an electromagnetic valve 14, a pressure supply pipe 15, and a micro-fluid chip 16.

In the present embodiment, the computer 11 is connected to the microscope 12 and the signal conversion interface 13. The computer 11 acquires image data output from the microscope 12. The computer 11 also outputs a digital signal, and the digital signal is input to the signal conversion interface 13. Details will be described later. The microscope 12 includes an image sensor capable of capturing an image included in its visual field. In the present embodiment, the microscope 12 can capture an image using a manipulation region 60 of the micro-fluid chip 16, which is described later, as the visual field. Image data indicating the captured image is transferred to the computer 11.

The signal conversion interface 13 is a device connected to the computer 11 and the electromagnetic valve 14 to output a predetermined current to the electromagnetic valve 14 according to the digital signal output from the computer 11. A compressor (not illustrated) and the pressure supply pipe 15 are connected to the electromagnetic valve 14, and connection between the compressor and the pressure supply pipe 15 is switched by opening and closing the electromagnetic valve 14. Therefore, the electromagnetic valve 14 can switch an internal pressure of the pressure supply pipe 15 between a high state and a low state. That is, the electromagnetic valve 14 adjusts the internal pressure of the pressure supply pipe 15 according to the current supplied from the signal conversion interface 13. In the present embodiment, the electromagnetic valve 14 sets the internal pressure of the pressure supply pipe 15 to the high state in a period in which the current output from the signal conversion interface 13 is at a high level, and sets the internal pressure of the pressure supply pipe 15 to the low state in a period in which the current is at a low level.

The pressure supply pipe 15 is connected to the micro-fluid chip 16. An air pressure adjustment unit 70 (described later) is formed on the micro-fluid chip 16. Therefore, the electromagnetic valve 14 can switch an internal pressure of the air pressure adjustment unit 70 to which the pressure supply pipe 15 is connected between a high state and a low state. In the present embodiment, six of the electromagnetic valves 14 and six of the pressure supply pipes 15 are provided as illustrated in Fig. 1A, one of which is denoted by a reference sign in Fig. 1A.

The micro-fluid chip 16 is provided with a liquid container containing liquid and a structure containing gas. Fig. 1B illustrates the liquid container and the structure provided on the micro-fluid chip 16. The micro-fluid chip 16 has a rectangular parallelepiped shape in which two surfaces are larger than the other four surfaces, that is, a plate shape, and the liquid container and the structure are formed on a surface having a larger area than the other surfaces. Fig. 1B illustrates the micro-fluid chip 16 as viewed from a direction perpendicular to the surface having a larger area than the other surfaces.

The micro-fluid chip 16 is provided with a first channel 40, a second channel 50, the manipulation region 60, and the air pressure adjustment unit 70. In the present embodiment, the liquid is water, and the gas is air. Although an object to be manipulated is introduced into the first channel 40 or the manipulation region 60, a configuration for introducing the object to be manipulated is omitted in Fig. 1B.

The first channel 40, the second channel 50, and the manipulation region 60 are channels and a region containing the liquid. The first channel 40 is a linear hollow space, one end of which is connected to the manipulation region 60. In addition, the other end of the first channel 40 is connected to the second channel 50. In the present embodiment, three of the first channels 40 are provided. In the present embodiment, the first channels 40 are evenly spaced apart in the largest plane of the micro-fluid chip 16 (a plane parallel to the paper surface of Fig. 1B). Therefore, in Fig. 1B, an angle between adjacent first channels 40 is 120°.

The manipulation region 60 is a region containing the liquid to manipulate disposition of the object to be manipulated at a predetermined position, and has an internal space in which the object to be manipulated is moved planarly. In the present embodiment, the manipulation region 60 and the like are formed on the largest surface of the micro-fluid chip 16, and the internal space of the manipulation region 60 has a shape that enables planar movement of the object to be manipulated in a direction parallel to the largest surface of the micro-fluid chip 16. That is, the manipulation region 60 has an internal space extending in the direction parallel to the largest surface of the micro-fluid chip 16 at a fixed depth (for example, a depth of 30 µm or less) in the largest surface.

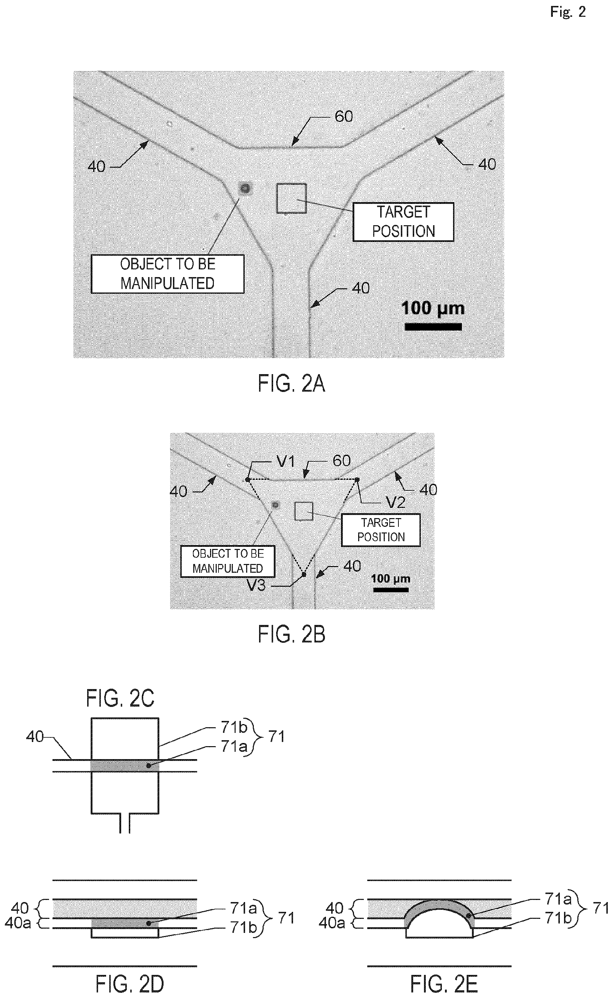

Furthermore, the internal space of the manipulation region 60 has a triangular shape as viewed from the direction perpendicular to the largest surface of the micro-fluid chip 16. Fig. 2A illustrates an example of an image including the manipulation region 60 captured by the microscope 12. The manipulation region 60 is a space surrounded by three sides. Assuming vertexes V1 to V3 intersecting by extending the three sides as indicated by broken lines in Fig. 2B, the manipulation region 60 has a triangular internal space having the vertexes V1 to V3. That is, in the present embodiment, extending inner walls of the internal space of the manipulation region 60 forms a triangle.

The first channels 40 are connected to vertex portions of the triangle formed by the manipulation region 60. In the present embodiment, the three first channels 40 are formed in directions substantially parallel to straight lines extending from the center of gravity toward the vertexes of the triangle formed by the manipulation region 60. In the present embodiment, the first channels 40 have the same depth as that of the manipulation region 60, and have a width smaller than one side of the triangle formed by the manipulation region 60. In the present embodiment, the first channels 40 are channels extending linearly. In the present embodiment, the first channels 40 are hollow spaces having a rectangular cross section. One end of each of the first channels is connected to the manipulation region 60 as illustrated in Fig. 2A, and the other end thereof is connected to the second channel 50 as illustrated in Fig. 1B. In the present specification, the depth is a length in the direction perpendicular to the largest surface of the micro-fluid chip 16, and the width is in a direction perpendicular to the depth direction and in a short direction.

As illustrated in Fig. 1B, the second channel 50 includes a storage part 51 and a coupling part 52. In the present embodiment, the storage part 51 has a circular shape as viewed from the direction perpendicular to the largest surface of the micro-fluid chip 16. The storage part 51 is connected to each first channel 40 and has a larger depth than that of the first channels 40 (for example, 3 mm to 5 mm). In addition, the diameter of the circle formed by the storage part 51 is larger than the width of the first channels 40. In the present embodiment, three of the storage parts 51 are provided, and all have the same depth and shape. In addition, in the present embodiment, the rectangular-parallelepiped micro-fluid chip 16 is placed on a horizontal plane such that the liquid levels of the three storage parts 51 have the same height.

The coupling part 52 is formed so as to couple two adjacent storage parts 51 together between the storage parts 51. In the present embodiment, the coupling part 52 is a hollow space having a rectangular cross section. The coupling part 52 has the same depth as that of the first channels 40. The width is smaller than the diameter of the circles formed by the storage parts 51. Therefore, the storage part 51 is larger than the coupling part 52**.** In the present embodiment, the storage parts 51 and the coupling parts 52 are present around the triangle formed by the manipulation region 60, the storage parts 51 correspond to the vertexes of a triangle larger than the manipulation region 60, and the coupling parts 52 correspond to the sides of the triangle larger than the manipulation region 60. Therefore, the second channel 50 can also be regarded as a looped channel formed so as to connect the storage parts 51 around the manipulation region 60.

The air pressure adjustment unit 70 is a structure including a passage containing the gas. The air pressure adjustment unit 70 includes a switching part 71, a gas passage 72, and a connection part 73. The connection part 73 is a portion to which the pressure supply pipe 15 is connected. In the present embodiment, six of the connection parts 73 are formed, and the six pressure supply pipes 15 are connected to the respective connection parts 73. The gas passage 72 is a hollow space that is connected to each connection part 73 to bring the connection part 73 into communication with a space immediately below the switching part 71 formed immediately below the first channel 40. Six of the air pressure adjustment units 70 are provided as illustrated in Fig. 1B, one of which is denoted by a reference sign in Fig. 1B.

The switching part 71 includes a mechanism capable of switching the first channel 40 between a closed state and a non-closed state. In the present embodiment, two of the switching parts 71 are provided in each of the three first channels 40. Figs. 2C to 2E are diagrams for explaining the structure of the switching part 71 and adjustment of an internal pressure of the first channel 40 by the switching part 71. Fig. 2C illustrates the switching part 71 as viewed from the direction perpendicular to the largest surface of the micro-fluid chip 16. Figs. 2D and 2E illustrate the switching part 71 as viewed from the direction parallel to the largest surface of the micro-fluid chip 16.

In the present embodiment, the switching part 71 includes a diaphragm 71a, and a space 71b is provided immediately below the diaphragm 71a. The first channel 40 and the space 71b are separated by a flexible thin film 40a, and in the thin film 40a, a portion where the first channel 40 and the space 71b overlap each other is the diaphragm 71a.

The dark gray illustrated in Figs. 2C to 2E is the diaphragm 71a, and the light gray illustrated in Figs. 2D and 2E is the liquid contained in the first channel 40. The space 71b is a rectangular parallelepiped cavity connected to the gas passage 72. The diaphragm 71a is not limited to a particular material, but is made of resin in the present embodiment.

As described above, the diaphragm 71a is a portion where the first channel 40 and the space 71b overlap each other, and has a rectangular shape as viewed from the direction perpendicular to the largest surface of the micro-fluid chip 16.

When the internal pressure of the pressure supply pipe 15 becomes high or low, an internal pressure of the space 71b also becomes high or low. Since the diaphragm 71a is a flexible film, its shape changes according to the internal pressure of the space 71b. Fig. 2D illustrates the shape of the space 71b in a state in which the internal pressure is low. Fig. 2E illustrates the shape of the space 71b in a state in which the internal pressure is high. As illustrated in Fig. 2E, when the internal pressure of the space 71b becomes high, the diaphragm 71a swells toward the first channel 40 and closes the first channel 40. That is, the first channel 40 comes into a closed state. As illustrated in Fig. 2D, when the internal pressure of the space 71b becomes low, the diaphragm 71a becomes flat, enabling the liquid to flow in the first channel 40. That is, the first channel 40 comes into an open state.

As described above, in a case where the first channel 40 is switched between the closed state and the non-closed state, the internal pressure of the first channel 40 fluctuates during the change, and the liquid flows. For example, when the state illustrated in Fig. 2D is switched to the state illustrated in Fig. 2E, the liquid flows through the first channel 40 toward the left side and the right side. In the present embodiment, the two switching parts 71 are provided in the single first channel 40. Therefore, by combining the operations of the two switching parts 71, the liquid can be moved from the first channel 40 to the manipulation region 60, and the liquid can be moved from the manipulation region 60 to the first channel 40.

In the present embodiment, one switching part 71 can realize two states of the state in which the first channel 40 is closed and the state in which the first channel 40 is not closed. Furthermore, in the present embodiment, since the two switching parts 71 are provided in each of the three first channels 40, a total of six switching parts 71 are provided. By associating 1 with the state in which the first channel 40 is closed and 0 with the state in which the first channel 40 is not closed, a state realized by the total of six switching parts 71 can be expressed by a 6-bit number. Hereinafter, the state realized by the total of six switching parts 71 and expressed by a 6-bit number is referred to as a state of the switching parts 71.

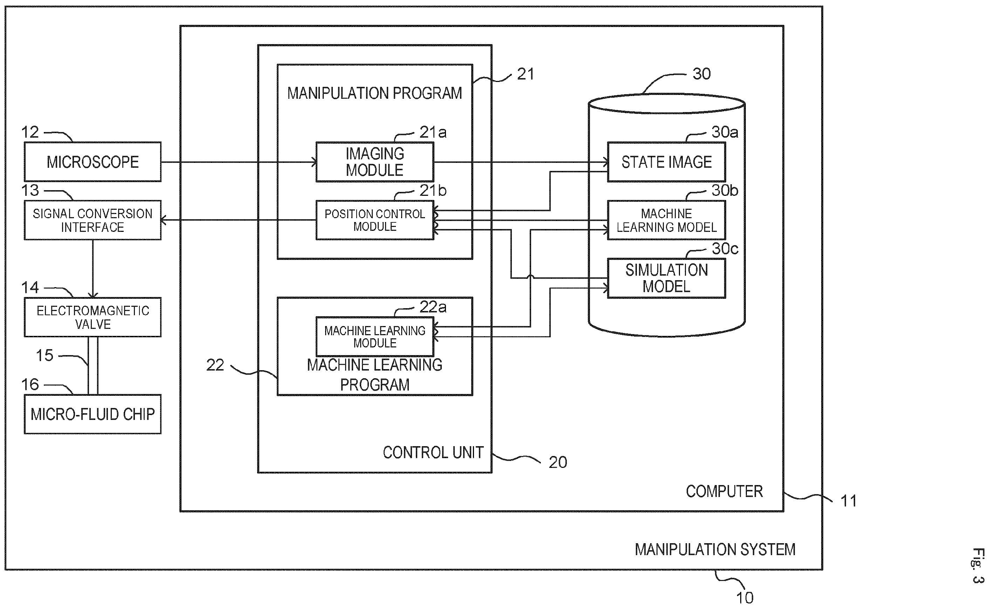

In the present embodiment, the object to be manipulated can be moved to a predetermined target position in the manipulation region 60 by controlling the above-described device by the computer 11. Fig. 3 is a diagram for explaining a configuration of the computer 11. The computer 11 includes a control unit 20 and a storage medium 30. The control unit 20 includes a CPU, a RAM, a ROM, and a GPU (not illustrated), and can execute various programs stored in the storage medium 30 or the like. The control unit 20 and the storage medium 30 may be configured by an integrated computer, or may be configured such that at least a part thereof is a separate device and is connected by various cables or the like. Of course, an output unit that outputs an image, a sound, or the like, an input unit that allows a user to input an instruction or the like, and the like may be provided. The output unit and the input unit may be connectable via various interfaces.

In the present embodiment, the control unit 20 can execute a manipulation program 21 and a machine learning program 22. The manipulation program 21 is a program that causes the control unit 20 to execute a function of moving the object to be manipulated to the target position on the basis of the image of the manipulation region 60 captured by the microscope 12. When the manipulation program 21 is executed, the control unit 20 functions as an imaging module 21a and a position control module 21b.

In the present embodiment, the movement of the object to be manipulated is executed on the basis of a result of machine learning performed in advance. The machine learning program 22 is a program that causes the control unit 20 to execute a function of machine-learning a model for determining a control target for the switching parts 71 on the basis of the image of the manipulation region 60 captured by the microscope 12 or the like. When the machine learning program 22 is executed, the control unit 20 functions as a machine learning module 22a.

### (2) Machine Learning Process Using Artificial Intelligence (AI) :

In the present embodiment, the model for determining the control target for the switching parts 71 is trained with machine-learning. In order to support the machine learning, a model for simulating the movement of the object to be manipulated by the liquid is also trained with machine-learning. Here, the former is simply referred to as a machine learning model 30b, and the latter is referred to as a simulation model 30c.

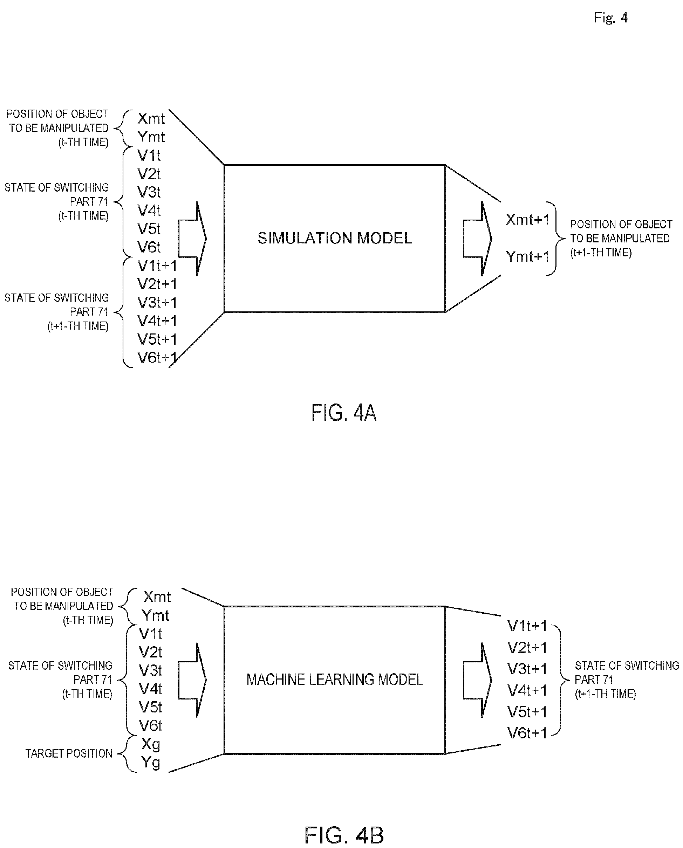

In the present embodiment, the simulation model 30c uses, as input data, information indicating the position of the object to be manipulated and the current states and the next states of the switching parts 71, and outputs, as output data, information indicating the next position of the object to be manipulated. Fig. 4A is a diagram schematically illustrating the simulation model 30c. In Fig. 4A, the input data is illustrated on the left side, the output data is illustrated on the right side, and when the input data is input to the simulation model 30c, the output data is output from the right side. In Fig. 4A, Xm, Ym indicates the position (coordinates) of the object to be manipulated. V1 to V6 indicate the respective states (1 or 0) of the six switching parts 71. V1 to V6 are lined up to obtain the 6-bit number indicating the state of the switching parts 71.

A sign t is added to the sign indicating the position of the object to be manipulated and the states of the switching parts 71. The sign t is a sign for identifying the number of operations by the switching parts 71. Therefore, as illustrated in Fig. 4A, the position of the object to be manipulated after a t-th operation is input to the simulation model 30c. In addition, the states of the switching parts 71 after the t-th operation and the states of the switching parts 71 after a t+1-th operation are input to the simulation model 30c. Then, the position of the object to be manipulated after the t+1-th operation is output. Therefore, according to the simulation model 30c, in a case where the position of the object to be manipulated is a specific position and the switching parts 71 are in a specific state after the t-th operation, it is possible to simulate to which position the object to be manipulated is displaced by subsequently changing the switching parts 71 to a specific state.

This simulation model 30c can be trained with machine-learning by various methods. For example, the simulation model 30c can be generated by optimizing a model configured by a neural network by machine learning. Specifically, in a case where the switching parts 71 are in a specific state, the microscope 12 in the manipulation system 10 performs imaging, and the position (coordinates) of the object to be manipulated is specified. In this state, the computer 11 outputs a signal to the signal conversion interface 13 to change the states of the switching parts 71.

Then, the microscope 12 performs imaging again, and the position of the object to be manipulated according to the state change is specified. By collecting the information on the series of operations, the position of the object to be manipulated after the state change can be associated with the information indicating the current position of the object to be manipulated and the current states and the next states of the switching parts 71. This set is used as training data, and a sufficient number of training data samples for learning a neural network are collected. After the training data is collected, the control unit 20 executes a known machine learning process by the function of the machine learning module 22a to optimize the neural network. The model thus obtained is the simulation model 30c and is recorded in the storage medium 30.

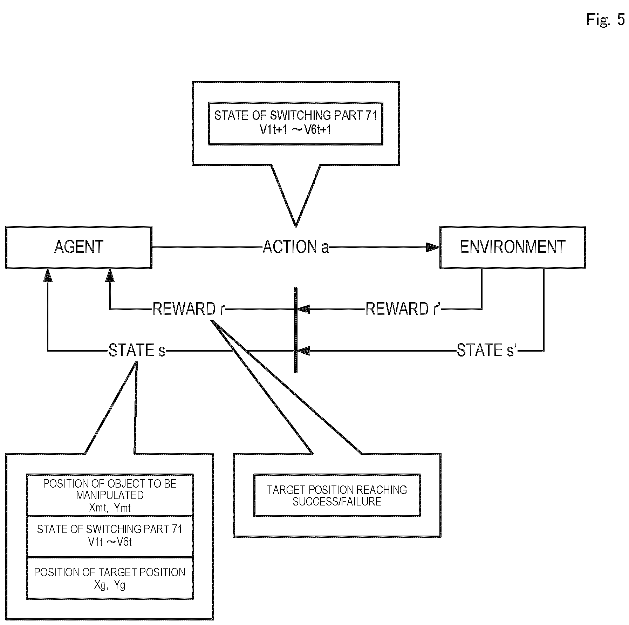

The machine learning of the machine learning model 30b in an environment is performed in a state in which the simulation model 30c has already been created. The machine learning of the machine learning model 30b may also be performed by various methods. Here, an example of machine-learning the machine learning model 30b by reinforcement learning will be described. Fig. 5 is a diagram for explaining learning of the machine learning model 30b by a model of reinforcement learning including an agent and an environment. The agent illustrated in Fig. 5 corresponds to a function of selecting an action a that can be taken in a current state s. The environment is used to determine a next state s' on the basis of the action a selected by the agent and the current state s, and determine a reward r' on the basis of the action a, the state s, and the state s'. The environment only needs to be defined so as to be able to determine the next state s' from the action a and the state s and further determine the reward r'. Therefore, the state and the like may be actually observed, or the state and the like may be determined in a virtual environment. In the present embodiment, the virtual environment is defined, for example, by virtually reproducing the manipulation region 60 in a three-dimensional space virtually provided in the computer 11. The position of the object to be manipulated and the like in the virtual three-dimensional space are input to the simulation model 30c, whereby a state change under the virtual environment is simulated. The reinforcement learning is performed under the virtual environment using the simulation model 30c as described above, which makes it possible to realize optimization of the machine learning model 30b at a higher speed than in an environment requiring actual observation. In a case where the machine learning model 30b is optimized under the virtual environment and the machine learning of the machine learning model 30b is further performed after an operation start of the manipulation system 10 using the machine learning model 30b, information observed under a real environment may be used.

Various methods can be adopted as a reinforcement learning algorithm, and here, Q-learning will be described as an example. In the Q-learning, an action-value function Q(s, a) is assumed that indicates expected return in a case where the action a is selected according to the current state s and then the action a is selected according to a predetermined policy. When the action-value function Q(s, a) is optimized, an optimal policy is obtained. The policy may be defined in various modes, and for example, a greedy policy is a policy in which an action that maximizes the action-value function Q(s, a) is selected. When the action-value function Q(s, a) is optimized in a state in which such a policy is assumed, the policy of selecting the action a so as to maximize the action-value function Q(s, a) in the state s is the optimal policy. The machine learning model 30b may be defined in various modes. Here, the machine learning model 30b is defined such that data including the action-value function Q(s, a) and indicating the action a that maximizes the action-value function Q(s, a) is output data.

In the present embodiment, the state s is the position of the object to be manipulated, the states of the switching parts 71, and the target position of the object to be manipulated. The action a is operations in the switching parts 71 necessary for bringing the object to be manipulated close to the target position, that is, the next states of the switching parts 71. Therefore, the machine learning model 30b is defined so as to use information indicating the position of the object to be manipulated, the states of the switching parts 71, and the target position of the object to be manipulated as input data, and output information indicating the operations in the switching parts 71 necessary for bringing the object to be manipulated close to the target position as output data.

Fig. 4B is a diagram schematically illustrating the machine learning model 30b. Reference signs are denoted in a similar manner to that of Fig. 4A. In addition, Xg, Yg is the position (coordinates) of the target position of the object to be manipulated. The position is coordinates in a virtually constructed environment under the virtual environment, and is a position in an image captured by the microscope 12 under the real environment. As illustrated in Fig. 4B, the position of the object to be manipulated at the t-th time, the states of the switching parts 71 after the t-th operation, and the target position of the object to be manipulated are input to the machine learning model 30b. These positions and states input to the machine learning model 30b correspond to the current state s illustrated in Fig. 5. The output from the machine learning model 30b is the states of the switching parts 71 after the t+1-th operation and corresponds to the action a illustrated in Fig. 5. Therefore, assuming that the t-th time is the current time, the machine learning model 30b outputs the next action a (the next states of the switching parts 71) on the basis of the current state s (the current states and the target position of the object to be manipulated and the switching parts 71).

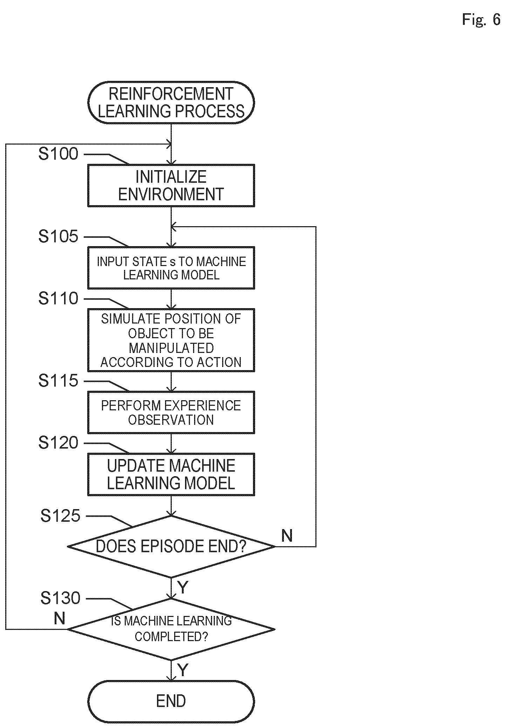

Fig. 6 is a flowchart illustrating a main part of a reinforcement learning process for generating the machine learning model 30b as described above. In Fig. 6, a process of machine-learning the machine learning model 30b under the virtual environment will be described. The reinforcement learning process illustrated in Fig. 6 is performed in a state in which information indicating the optimized simulation model 30c, the non-optimized machine learning model 30b, and the virtually constructed environment is recorded in the storage medium 30. When the reinforcement learning process is started, the control unit 20 initializes the virtual environment constructed for learning by the function of the machine learning module 22a (step S100). That is, the control unit 20 sets the value of a variable or the like to be used in the course of the reinforcement learning process to an initial value. Specifically, the control unit 20 sets the position of the object to be manipulated and the initial state of the switching parts 71, and further virtually sets the target position. The initial value of the virtual position of the object to be manipulated is an initial position in learning, and a position virtually determined in advance is the initial position. There may be a plurality of initial positions depending on the nature of a task, and the respective initial positions are processed in each episode repeated in learning loop processing of steps S100 to S130.

The virtual target position can be set in the virtually reproduced manipulation region 60, and any position in the manipulation region 60 can be set as the virtual target position. In each episode repeated in the loop processing of steps S100 to S130, the target position may be changed or fixed. In a case where the manipulation system 10 moves the object to be manipulated toward a specific target position, it is preferable that the target position is fixed to the specific target position within the same episode (S100 to S125). In a case where the target position is not limited to a specific target position, and a plurality of positions can be set as the target position, it is preferable that machine learning is performed with the plurality of positions as the initial values of the target position also in the reinforcement learning process. During repetition of the loop processing of steps S100 to S130, the initial value of the state of the switching parts 71 may be fixed to a specific state (for example, 000000 or the like) or may change.

Next, the control unit 20 inputs the state s to the machine learning model 30b by the function of the machine learning module 22a (step S105). That is, the control unit 20 inputs the current state (the position Xmt, Ymt of the object to be manipulated, the states V1t to V6t of the switching parts 71, and the target position Xg, Yg) to the machine learning model 30b being trained. As a result, the states V1t+1 to V6t+1 of the switching parts 71 are output as the next action. In the course of the machine learning, the next states V1t+1 to V6t+1 of the switching parts 71 are not necessarily appropriate, but are gradually optimized as the machine learning progresses.

Next, the control unit 20 simulates the position of the object to be manipulated according to the action by the function of the machine learning module 22a (step S110). That is, the control unit 20 inputs the position Xmt, Ymt of the object to be manipulated, the current states V1t to V6t of the switching parts 71, and the next states V1t+1 to V6t+1 of the switching parts 71 obtained in step S105 to the simulation model 30c recorded in the storage medium 30. As a result, the next position Xmt+1, Ymt+1 of the object to be manipulated is output. This makes it possible to reproduce a movement phenomenon of the object to be manipulated equivalent to the real environment in the virtual environment without using the real environment.

Next, the control unit 20 performs experience observation by the function of the machine learning module 22a (step S115). That is, the control unit 20 specifies a state and a reward corresponding to the action in steps S105 and S110. Specifically, the control unit 20 specifies the reward on the basis of the target position Xg, Yg and the next position Xmt+1, Ymt+1 of the object to be manipulated. The reward may be determined by various methods. Specifically, the reward is defined so as to obtain a positive reward in a case where a distance between the target position Xg, Yg and the next position Xmt+1, Ymt+1 of the object to be manipulated is within a predetermined distance. In addition to this, for example, a larger reward may be added as the distance between the target position Xg, Yg and the next position Xmt+1, Ymt+1 of the object to be manipulated is smaller, or a negative reward having a larger absolute value as the distance is larger may be added. In any case, when the reward is defined, the action-value function Q(s, a) can be defined by using a known definition, for example.

Next, the control unit 20 updates the machine learning model 30b by the function of the machine learning module 22a (step S120). The machine learning model 30b includes the action-value function Q(s, a), and aims to optimize the action-value function Q(s, a) that is not optimized at an initial stage in the reinforcement learning. In the present embodiment, the action-value function Q(s, a) is defined by a multilayer neural network, and the multilayer neural network is optimized in the course of the reinforcement learning. The optimization of the multilayer neural network may be performed by various known algorithms, and for example, it is possible to adopt a configuration for optimizing the multilayer neural network by an objective function that minimizes a temporal difference (TD) error.

When the action-value function Q(s, a) is updated according to a known algorithm, that is, the machine learning model 30b is updated, the control unit 20 determines whether or not the process of the current episode has ended by the function of the machine learning module 22a (step S125). In the present embodiment, one episode is from the first observation of the position of the object to be manipulated until the object to be manipulated reaches the target position Xg, Yg or until the object to be manipulated fails to reach the target position Xg, Yg. A failure condition only needs to be determined in advance. For example, it is possible to adopt a configuration in which the failure condition is considered to be satisfied in a case where the state s is changed a predetermined number of times or more without the object to be manipulated reaching the target position Xg, Yg. In addition, for example, a configuration in which the failure condition is considered to be satisfied in a case where the object to be manipulated reaches the outside of the manipulation region 60 may be adopted.

In the case of not determining that the process of the current episode has ended in step S125, the control unit 20 repeats the process from step S105. In the case of determining that the process of the current episode has ended in step S125, the control unit 20 determines whether or not the machine learning has been completed (step S130). A completion condition of the machine learning may be determined in various modes. For example, it is possible to adopt a configuration in which the completion condition of the machine learning is considered to be satisfied in a case where the process of steps S105 to S125 is completed for all of a plurality of environments prepared in advance. In addition, an index for evaluating whether or not the machine learning model 30b is sufficiently optimized, for example, a task achievement rate may specify the satisfaction of the completion condition.

In the case of not determining that the machine learning has been completed in step S130, the control unit 20 repeats the process from step S100 by the function of the machine learning module 22a. In the present embodiment, at this time, the environment is initialized so as to obtain an initial state different from the previous episode. In the case of determining that the machine learning has been completed in step S130, the control unit 20 ends the reinforcement learning process. As a result, the optimized machine learning model 30b is obtained.

### (3) Manipulation Process:

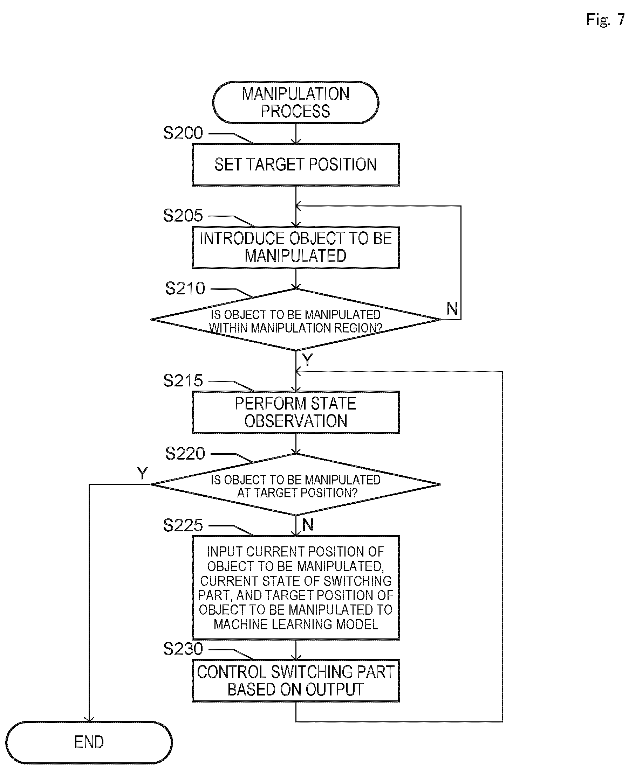

Next, a manipulation process for manipulating disposition of the object to be manipulated at the target position will be described. Fig. 7 is a flowchart illustrating the manipulation process. The manipulation process is realized by the control unit 20 executing the manipulation program 21 in a state in which the optimized machine learning model 30b is recorded in the storage medium 30. When the manipulation program 21 is executed, the control unit 20 functions as the imaging module 21a and the position control module 21b. The imaging module 21a is a function of outputting a control signal to the microscope 12 and acquiring image data indicating an image in the visual field of the microscope 12. The position control module 21b is a function of outputting a control signal to the signal conversion interface 13 and causing the signal conversion interface 13 to output a current.

A user instructs the manipulation system 10 to start the manipulation process using the input unit (not illustrated) or the like. When the manipulation process is started, the control unit 20 receives the setting of the target position (step S200). That is, the control unit 20 acquires coordinates input by the user using the input unit (not illustrated) or the like, and regards the coordinates as the target position in the manipulation region 60.

Next, the control unit 20 introduces the object to be manipulated by the function of the position control module 21b (step S205). That is, the user introduces the object to be manipulated into the first channel 40, the second channel 50, or the manipulation region 60 by manipulating an unillustrated configuration. The control unit 20 controls the signal conversion interface 13 and operates the switching parts 71 by the function of the position control module 21b to perform a process for moving the object to be manipulated into the manipulation region 60. The process for moving the object to be manipulated into the manipulation region 60 is, for example, a process for moving the object to be manipulated in the first channel 40 toward the manipulation region 60, and the operations required in the switching parts 71 are simple operations determined in advance.

Next, the control unit 20 determines whether or not the object to be manipulated is present in the manipulation region by the function of the imaging module 21a (step S210). That is, the control unit 20 controls the microscope 12 to acquire image data, and extracts the object to be manipulated from the image data. Then, the control unit 20 determines whether or not the position of the object to be manipulated is within the manipulation region 60. In the case of not determining that the object to be manipulated is present within the manipulation region, the control unit 20 repeats the process from step S205. However, in the case where step S205 is repeated, the process for moving the object to be manipulated toward the manipulation region 60 is performed instead of newly introducing the object to be manipulated.

In the case of determining that the object to be manipulated is present within the manipulation region in step S210, the control unit 20 performs the process from step S215. The state in which it is determined that the object to be manipulated is present within the manipulation region is, for example, the state illustrated in Fig. 2A. In Fig. 2A, a rectangular frame is added to the image of the object to be manipulated, and a rectangular frame is added to the target position. However, these rectangular frames are superimposed on the image and are not objects existing in the manipulation region 60.

In step S215, the control unit 20 performs state observation. That is, the control unit 20 controls the microscope 12 to acquire image data, and acquires the current position (Xmt, Ymt) of the object to be manipulated from the image data by the function of the imaging module 21a. In addition, the control unit 20 outputs a control signal to the signal conversion interface 13 and acquires the current states (V1t to V6t) of the switching parts 71. Furthermore, the control unit 20 acquires the target position (Xg, Yg) set in step S200.

Next, the control unit 20 determines whether or not the object to be manipulated has reached the target position (step S220). That is, the control unit 20 determines that the object to be manipulated has reached the target position in a case where the current position (Xmt, Ymt) of the object to be manipulated acquired in step S215 is within a predetermined distance from the target position (Xg, Yg) set in step S200. In the case of determining that the object to be manipulated has reached the target position in step S220, the control unit 20 ends the manipulation process.

In the case of not determining that the object to be manipulated has reached the target position in step S220, the control unit 20 inputs the position (Xmt, Ymt) of the object to be manipulated, the current states (V1t to V6t) of the switching parts 71, and the target position (Xg, Yg) of the object to be manipulated to the machine learning model 30b by the function of the position control module 21b (step S225). That is, the control unit 20 inputs the current state to the machine learning model 30b. As a result, the machine learning model 30b outputs the next action, that is, the states (V1t+1 to V6t+1) of the switching parts 71 to be set next.

The control unit 20 controls the switching parts 71 on the basis of the output by the function of the position control module 21b (step S230). That is, the output of the machine learning model 30b is a 6-bit number indicating the state of the switching parts 71 to be set next. Therefore, the control unit 20 controls the signal conversion interface 13 so as to supply a current to the electromagnetic valve 14 corresponding to a numerical value 1 and stop current supply to the electromagnetic valve 14 corresponding to a numerical value 0. As a result, the air pressure is adjusted by the six electromagnetic valves 14, the diaphragms 71a of the switching parts 71 operate by the pressure supply pipes 15, and the states of the first channels 40 change. As a result, the liquid in the first channels 40 moves, and the object to be manipulated in the manipulation region 60 moves due to the movement of the liquid. In this manner, in the present embodiment, the air pressure adjustment units 70, the imaging module 21a, the position control module 21b, the signal conversion interface 13, the electromagnetic valves 14, and the pressure supply pipes 15 constitute a liquid control unit.

After step S230 is executed, the control unit 20 repeats the process from step S215. According to the above process, the control unit 20 can move the object to be manipulated to the target position by repeating the state observation and the state input to the machine learning model 30b. The machine learning model 30b outputs the next states of the switching parts 71 necessary for movement to the target position with the current state input thereto. By repeating such control of the position of the object to be manipulated, the object to be manipulated reaches the target position. As long as the machine learning model 30b is properly trained with machine-learning, this process can be realized by the simple process of the state observation and the state input, and the object to be manipulated can be accurately moved to the target position. Therefore, a task that is typically very difficult, that is, the movement of the object to be manipulated by switching the states of the switching parts 71 can be achieved very easily and accurately.

Since the first channels 40 are provided with the total of six switching parts 71, the state that can be realized by the switching parts 71 is any of 64 states that can be expressed by 6 bits. The machine learning model 30b repeats the process of selecting the next state from the 64 states according to the current state and switching the switching parts 71 so as to obtain the next state, so that the object to be manipulated moves to the target position in the manipulation region 60. In the 64 states, in each of the first channels 40, movement of the liquid from the first channel 40 toward the manipulation region 60, movement of the liquid in a direction opposite to the movement, movement of the liquid from the first channel 40 toward the second channel 50, and movement of the liquid in a direction opposite to the movement can occur. The movement of the liquid occurs immediately after the state change of the switching parts 71, and the pressure fluctuates at this time. If the pressure fluctuation remains until the next state change, the movement of the liquid due to the next state change becomes different from the movement originally assumed. Therefore, during repetition of the state change of the switching parts 71, the moving direction and the moving amount of the liquid become different from those originally assumed, and as a result, it becomes difficult to control the position of the object to be manipulated.

However, in the present embodiment, as illustrated in Fig. 1B, the plurality of first channels 40 are connected to the manipulation region 60, and the second channel 50 is connected to all the first channels 40. In the present embodiment, the second channel 50 is a series of channels surrounding the first channels 40. That is, the plurality of first channels 40 are connected to each other through the second channel 50. With such a configuration, in the present embodiment, the pressures in the first channels 40 are easily made uniform by the second channel 50. Therefore, a local pressure difference is less likely to occur in the first channels 40 and the manipulation region 60. In addition, even if the pressure fluctuates due to the movement of the liquid in a specific first channel 40, the pressure fluctuation is made uniform at an early stage. Therefore, in a case where the position of the object to be manipulated is manipulated by repeating the movement of the liquid, it becomes easy to control the movement of the liquid for manipulating the object to be manipulated. Furthermore, in the present embodiment, the switching parts 71 are provided in the first channels 40 and are not provided in the second channel 50. Therefore, the operations of the switching parts 71 necessary for manipulating the position of the object to be manipulated and the uniformization of the pressures in the first channels 40 can be performed at different places. Therefore, the operations of the switching parts 71 do not hinder the pressure uniformization.

Furthermore, in the present embodiment, the first channels 40 are thinner than the second channel 50. Specifically, the diameter of the circles formed by the storage parts 51 constituting the second channel 50 is 10 times or more larger than the width of the first channels 40 and several times or more larger than the width of the coupling parts 52. Furthermore, the width of the coupling parts 52 is several times larger than the width of the first channels 40.

Therefore, the storage parts 51 are much larger than the first channels 40 and the coupling parts 52. This makes it possible for the storage parts 51 to effectively absorb the pressure fluctuations occurring in the first channels 40 to uniformize the pressures in the first channels 40 at an early stage. In addition, since the storage parts 51 are connected together by each coupling part 52, different pressure fluctuations occurring in the plurality of first channels 40, if any, can be effectively absorbed at an early stage and the pressures in the first channels 40 can be made uniform. As described above, the second channel 50 functions as a very effective buffer against the pressure fluctuations occurring in the first channels 40 and the movement of the liquid. Therefore, the second channel 50 eliminates and uniformizes the pressure fluctuations in the first channels 40 and the manipulation region 60 at an early stage, and obtains an equilibrium state at an early stage. Therefore, it is possible to shift to the next state output by the machine learning model 30b at an early stage. In addition, it is possible to facilitate control of the movement of the liquid in the case of shifting to the next state.

Furthermore, the second channel 50 includes the coupling parts 52 and the storage parts 51 having a diameter larger than the width of the coupling parts 52. Moreover, the first channels 40 are connected to the storage parts 51. Therefore, the storage parts 51 larger than the first channels 40 and the coupling parts 52 effectively function as a buffer for uniformizing the pressure fluctuations occurring in the first channels 40 and the coupling parts 52 at an early stage. Furthermore, by coupling the storage parts 51 by each coupling part 52, a pressure difference generated between the plurality of first channels 40 is also uniformized at an early stage. The coupling parts 52 have a linear structure coupling the storage parts 51. The coupling parts 52 having a linear shape can couple the storage parts 51 with a distance as short as possible, which makes it possible to efficiently uniformize a pressure difference between the storage parts 51.

Furthermore, the manipulation region 60 has the internal space in which the object to be manipulated is moved planarly in the direction parallel to the largest surface of the micro-fluid chip 16. Therefore, in the present embodiment, it is sufficient that a substantially two-dimensional direction is assumed as the moving direction of the object to be manipulated, and the position of the object to be manipulated can be easily specified and easily controlled. Furthermore, the internal space of the manipulation region 60 viewed from the direction perpendicular to the largest surface of the micro-fluid chip 16 has a triangular shape. Therefore, the internal space has an extremely simple shape, and the manipulation region 60 can be easily formed.

Furthermore, the first channels 40 are connected to the vertexes of the triangle formed by the manipulation region 60. Therefore, in a case where the liquid flows into the manipulation region 60 from the first channels 40 or where the liquid flows out from the manipulation region 60 to the first channels 40, liquid stagnation or vortex is less likely to occur in the manipulation region 60. On the other hand, if the first channels 40 are connected to the sides of the triangle instead of the vertexes, liquid stagnation or vortex is likely to occur in the manipulation region 60. Therefore, in the present embodiment, it is easy to control the position of the object to be manipulated as compared with the configuration in which the first channels 40 are connected to the sides of the triangle.

Furthermore, in the above-described embodiment, the two switching parts 71 are provided in the single first channel 40. Therefore, the liquid can flow in and out between the first channels 40 and the manipulation region 60 independently in each of the three first channels 40 or by a combination of the three first channels 40. This makes it possible to easily control the position of the object to be manipulated in the manipulation region 60.

### (4) Other Embodiments:

The above embodiment is an example for carrying out the present invention, and various other embodiments can be adopted. For example, the shape and configuration of the second channel 50 are not limited to those of the above-described embodiment. Therefore, a mode may be adopted in which a fixed-width channel having a shape similar to the shape of the manipulation region 60 (for example, a polygon) and a larger size than the manipulation region 60 is formed around the manipulation region 60 and the first channels 40 to constitute the second channel 50. In addition, one large storage part may be formed around the manipulation region 60 and the first channels 40 to constitute the second channel. Various modes may be adopted. Furthermore, in a case where the second channel 50 includes the storage parts 51 and the coupling parts 52, the storage parts 51 may have a shape other than the circle.

Furthermore, the simulation of the position of the object to be manipulated according to the action in step S110 may be realized in a mode not using the simulation model 30c. For example, simulations by other methods, finite element analysis, various fluid simulations, simulations using molecular dynamics, or the like may be used.

Furthermore, the input and output modes of the machine learning model 30b are not limited to the above-described example. For example, the state of the switching parts 71 is not limited to the mode expressed by 6 bits, and may be expressed by a 1-digit number in the decimal number system (such as any numerical value of 0 to 5). In addition, the position of the object to be manipulated at the t+1-th time, output from the simulation model 30c, may be expressed by a displacement vector or the like from the current position, instead of coordinates. Furthermore, the target position may be defined by a wide region, instead of one point.

Furthermore, the state of one switching part 71 may be multiple states or may change continuously, instead of two states. Moreover, the machine learning model 30b may be updated during the operation process. For example, the simulation model 30c may be updated on the basis of data accumulated in the operation process, and the reinforcement learning may be executed using the updated simulation model 30c so as to increase accuracy as the operation progresses. At this time, the environment for specifying the state or the like may be a real environment, and in this case, the coordinates or the like of the object to be manipulated under the real environment are observed. Furthermore, the moving direction of the object to be manipulated is not limited to the two-dimensional direction, and the position control may be performed in a three-dimensional direction. In this case, the microscope 12 performs imaging from a plurality of different directions.

Furthermore, the reinforcement learning method is not limited to the Q-learning as described above, and a method such as SARSA may be used. In addition, a method in which a policy model and an action-value function model are separately modeled, for example, an Actor-Critic algorithm may be used. Furthermore, the machine learning model that outputs the next states of the switching parts 71 is not limited to the configuration generated by the reinforcement learning, and may be generated by a neural network or the like. The machine learning mode is not limited to the above-described embodiment, and the machine learning may be performed by appropriately selecting various elements such as the number of layers and the number of nodes constituting a model, the type of an activation function, the type of a loss function, the type of gradient descent, the type of an optimization algorithm of gradient descent, the presence or absence of mini-batch learning and the number of batches, a learning rate, a discount rate, an initial value, the type and the presence or absence of an overfitting suppression method, the presence or absence of a convolution layer, the size of a filter in a convolution operation, the type of the filter, the type of padding or stride, the type and the presence or absence of a pooling layer, the presence or absence of a fully connected layer, and the presence or absence of a recursive structure. Of course, learning may be performed by another machine learning, for example, support vector machine, clustering, or the like. Furthermore, the machine learning may be performed by automatically optimizing the structure (for example, the number of layers, the number of nodes for each layer, and the like) of a model.

In addition, the manipulation system may be realized by a plurality of devices, or may be a system or the like in which the machine learning is performed by a server and the position control is performed by a client. Furthermore, at least some of the imaging module 21a, the position control module 21b, and the machine learning module 22a may be provided separately in a plurality of devices. Furthermore, some configurations of the above-described embodiment may be omitted, or the processing steps may be changed in the order or partially skipped.

The manipulation region may be any region that can contain the liquid and the object to be manipulated whose position in the liquid is manipulated. That is, it is sufficient that the manipulation region is a liquid storage region, and the position of the object to be manipulated is variable in the manipulation region. Since the position of the object to be manipulated is manipulated by being carried by the liquid, the liquid is present at least at the target position of the object to be manipulated and a portion serving as a path to approach the target position. Since the movable liquid is present in the manipulation region, the manipulation region has a three-dimensional structure capable of storing the liquid. However, the substantial moving direction of the object to be manipulated may be three-dimensional or does not need to be three-dimensional. That is, the object to be manipulated may move planarly along a predetermined plane as in the above-described embodiment, or may move one-dimensionally.

The liquid is not particularly limited, and examples thereof include water, physiological saline, blood, serum, phosphate buffer solution, Good's buffer such as HEPES, oil, magnetic fluid, polymer solution, and photocurable resin. In addition, the liquid may be a combination thereof. Water is not particularly limited, and can be appropriately selected according to the purpose, and examples thereof include industrial water, tap water, ion-exchanged water, ultrafiltered water, reverse osmosis water, distilled water (pure water), and ultrapure water.

The object to be manipulated may be any object that can move in the liquid, and can be appropriately selected. For example, any particle such as a latex particle, a silica particle, a magnetic particle, a cell, and a liposome can be the object to be manipulated. The object to be manipulated may also be a bead, a microparticle obtained by modifying a receptor such as an antibody or a nanocolloid, or the like. In addition, the object to be manipulated may be a moving object such as a sperm or a microorganism.

The object to be manipulated only needs to be manipulated so as to be disposed at a predetermined position in the liquid present in the manipulation region, and the predetermined position may be a target position determined in advance, a trajectory position determined in advance, or the like. In any case, it is sufficient that the position where the object to be manipulated is to be disposed is determined as the predetermined position before the movement.

The liquid and the object to be manipulated only need to be contained in the manipulation region when the position of the object to be manipulated is manipulated by the manipulation system, and the liquid and the object to be manipulated may be present outside the manipulation region before the manipulation starts. In addition, the state in which the liquid and the object to be manipulated are contained only needs to be a state in which the liquid and the object to be manipulated are present inside the manipulation region. For example, in the case of the liquid, the manipulation region and each channel may be filled with the liquid, or may partially contain air or the like.

The first channel is the plurality of channels connected to the manipulation region and containing the liquid. That is, at least two first channels containing the liquid are connected to the manipulation region. As a result, an inflow path of the liquid to the manipulation region and an outflow path of the liquid from the manipulation region are secured, and it is possible to manipulate the object to be manipulated along with the movement of the liquid. The first channels may have any channel shape and cross-sectional shape as long as the liquid can move inside the first channels. In order to prevent a pressure distribution from becoming complicated and stagnation from occurring, it is preferable to use a simple shape, for example, a channel extending linearly. In addition, a simple cross-sectional shape is also preferable, and for example, a linear channel having a circular or rectangular cross section and a constant system can be adopted. The number of first channels is not limited to 3 as in the above-described embodiment, and may be various numbers.

The liquid control unit only needs to be able to move the liquid in the first channels. Various configurations may be adopted as the configuration for moving the liquid in the first channels. The configuration for moving the liquid by directly applying a pressure to the liquid is not limited to the configuration in which the first channels are switched between the closed state and the non-closed state as described above. For example, various configurations for moving the liquid in the first channels may be adopted. The configuration for applying a force to the liquid is also not limited to the configuration in which the diaphragm is changed by the air pressure, and the force may be applied to the liquid by various actuators such as a piezoelectric element. In addition, when the force is applied to the liquid, a configuration in which the force is indirectly applied may be adopted. For example, a configuration may be adopted in which the force is applied to the deformable first channels to move the liquid in the first channels.

It is sufficient that the second channel is the series of channels containing the liquid, and is connected to each of the plurality of first channels. That is, the plurality of first channels only need to be connected to each other through the second channel. It is sufficient that the second channel is connected to each of the plurality of first channels, so that the liquid in the second channel and the liquid in the first channels join together, and as a result, a pressure difference between the first channels is made uniform. Therefore, the shape of the second channel is not limited. For example, in the above-described embodiment, the three coupling parts 52 are provided, and the second channel 50 forms a triangle. However, the second channel is not limited to such a configuration, and the coupling parts 52 may have a shape having only two sides of the triangle. In addition, the storage parts and the coupling parts 52 do not have to be clearly distinguished from each other. For example, the coupling parts 52 having a thickness equivalent to the diameter of the storage parts 51 may be formed so as to couple the storage parts 51.

The second channel only needs to be the series of channels, in which the second channel itself is continuous. In order to uniformize the pressure, it is preferable that the second channel forms a closed loop as in the above-described embodiment. However, the second channel may be a series of channels having an end. In any case, it is sufficient that the plurality of first channels separately connected to the manipulation region are connected to the second channel formed as a continuous channel, so that the pressures of the first channels are made uniform.

The internal space of the manipulation region only needs to have a polygonal shape as viewed from the direction perpendicular to the plane along which the object to be manipulated moves. Therefore, the manipulation region is not limited to the triangular shape as in the above-described embodiment, and may be a quadrangular shape or the like, or may be a polygonal shape having more vertexes. In addition, the polygonal shape only needs to be the shape of the internal space, that is, a space in which the object to be manipulated can move. Therefore, in a case where the outer shape of the manipulation region and the shape of the internal space are different, it is sufficient that the internal space has a polygonal shape. Furthermore, the internal space only needs to be a space in which the object to be manipulated can be present in the manipulation region. Therefore, the inside of the manipulation region may be partitioned by a fence-like or lattice-like structure through which the liquid passes but the object to be manipulated does not pass.

Furthermore, in the above-described embodiment, the two switching parts 71 are provided in the single first channel 40, but the number of first channels 40 and the number of switching parts 71 are not limited to such an example. For example, three or more switching parts 71 may be provided in the single first channel 40, or the number of switching parts 71 provided in each of the plurality of first channels 40 may be different in each first channel 40. As long as one or more switching parts 71 are provided in each of the first channels 40, and the total number of the switching parts 71 having been provided is the number of first channels 40 + 1 or more, the inflow of the liquid into the manipulation region 60 and the outflow of the liquid from the manipulation region 60 can be realized by a combination of the states of the switching parts 71. Configuring the switching parts 71, the total number of which is the number of first channels 40 + 1 or more, makes it possible to realize control of the position of the object to be manipulated in the manipulation region 60 with a small number. In addition, configuring the switching parts 71, the total number of which is the number of first channels 40 + 1 or more, makes it possible to realize more precise position control as the number of switching parts 71 increases.

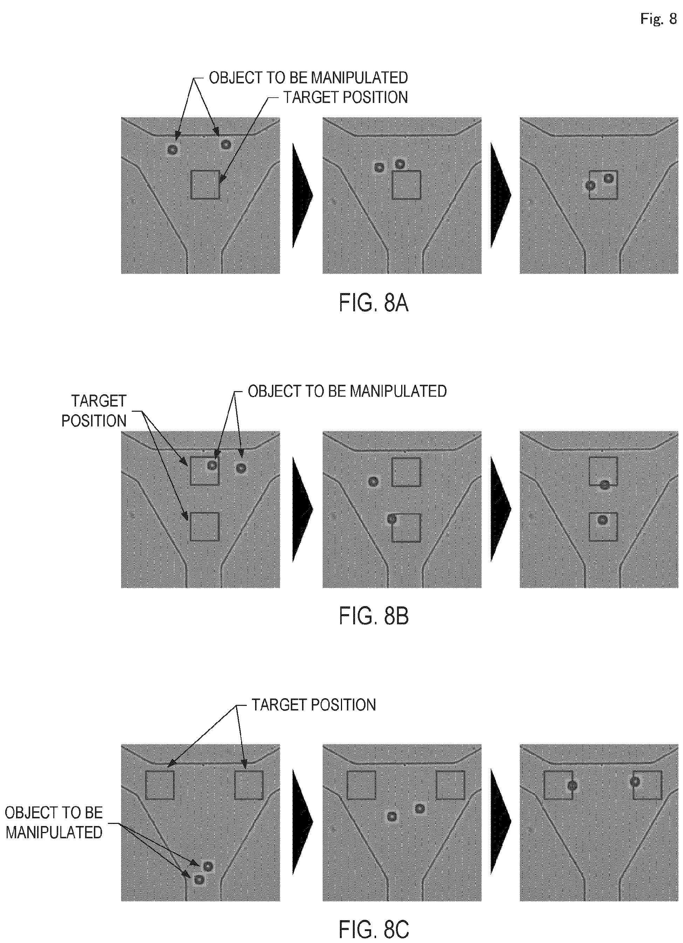

Furthermore, a plurality of target positions may be provided, or a plurality of objects to be manipulated may be provided. Fig. 8A illustrates images captured by the microscope 12 in an example in which there are two objects to be manipulated and there is one target position. Even in such a mode, by introducing the two objects to be manipulated into the manipulation region 60 and generating the machine learning model 30b by machine learning by setting the single target position, it is possible to perform control to manipulate the positions of the objects to be manipulated to reach the target position. In Fig. 8A, the image on the left side shows an initial state immediately after the objects to be manipulated are introduced, and the image at the center shows a state in which the objects to be manipulated are moving to the target position. In addition, the image on the right side shows a state in which the objects to be manipulated have reached the target position. As described above, it has been confirmed that the two objects to be manipulated can be moved to the single target position by machine learning.

Figs. 8B and 8C illustrate images captured by the microscope 12 in an example in which there are two objects to be manipulated and there are two target positions. Even in such a mode, by introducing the two objects to be manipulated into the manipulation region 60 and generating the machine learning model 30b by machine learning by setting the two target positions, it is possible to perform control to manipulate the positions of the objects to be manipulated to reach the target positions. Also in Figs. 8B and 8C, the image on the left side shows an initial state immediately after the objects to be manipulated are introduced, and the image at the center shows a state in which the objects to be manipulated are moving to the target positions. In addition, the image on the right side shows a state in which the objects to be manipulated have reached the target positions. As illustrated in these drawings, it has been confirmed that the two objects to be manipulated can be moved to the two target positions by machine learning. In addition, as illustrated in Figs. 8A to 8C, various combinations may be adopted as the number of objects to be manipulated, the number of target positions, and the initial position.

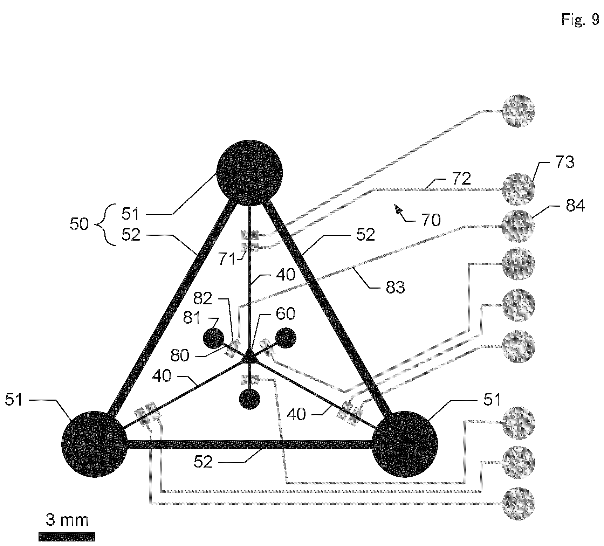

Fig. 9 is a diagram illustrating an example of a configuration for introducing an object to be manipulated into the first channel 40 or the manipulation region 60. In the example illustrated in Fig. 9, the configuration for introducing the object to be manipulated includes a third channel 80, a second storage part 81, a second switching part 82, a second gas passage 83, and a second connection part 84. In the example illustrated in Fig. 9, three sets of the third channels 80, the second storage parts 81, the second switching parts 82, the second gas passages 83, and the second connection parts 84 are formed, one set of which is denoted by reference signs in order to simplify the drawing.

The third channels 80 are channels connected to the sides of the triangle formed by the manipulation region 60 and extending in extending directions of the first channels 40. The third channels 80 contain the same liquid as the liquid in the manipulation region 60, and may have the same depth, width, and the like as those of the first channels 40. Each second storage part 81 is connected to one of opposite ends of the corresponding third channel 80 to which the manipulation region 60 is not connected. The second storage parts 81 have a diameter several times larger than the width of the third channels 80, contain the same liquid as the liquid in the manipulation region 60, and the object to be manipulated is stored in the liquid.

The second switching parts 82, the second gas passages 83, and the second connection parts 84 have the same configurations as the switching parts 71, the gas passages 72, and the connection parts 73. However, their installation positions are different. That is, the second switching parts 82 are formed immediately below the third channels 80, and the second gas passages 83 are passages connecting positions immediately below the second switching parts 82 and the second connection parts 84. The second connection parts 84 are connected to the electromagnetic valves 14. That is, in the example illustrated in Fig. 9, the number of electromagnetic valves 14 is 9 in total. The object to be manipulated is introduced by selecting the nine electromagnetic valves 14.

That is, the electromagnetic valves 14 switch internal pressures of the second gas passages 83 and the gas passages 72 between a high state and a low state. As a result, the third channels 80 and the first channels 40 are switched to either a closed state or a non-closed state. By using such state switching, the object to be manipulated stored in one of the three second storage parts 81 can be moved to the manipulation region 60. For example, only one of the three third channels 80 is brought into the non-closed state, and the other two third channels 80 are brought into the closed state. In this state, by operating any of the switching parts 71 to form a liquid flow from the manipulation region 60 to the first channels 40, the object to be manipulated stored in the second storage part 81 can be introduced into the manipulation region 60. According to the above configuration, the object to be manipulated can be easily introduced into the manipulation region 60.

According to the configuration illustrated in Fig. 9, the third channels 80, the second storage parts 81, and the like are disposed on the inner side of the second channel 50, so that the object to be manipulated stored near the manipulation region 60 can be introduced into the manipulation region 60. Therefore, the object to be manipulated moves only a short distance to be introduced, and the object to be manipulated can be easily introduced into the manipulation region 60. Furthermore, in the configuration illustrated in Fig. 9, the plurality of second storage parts 81 are provided. Therefore, different objects to be manipulated can be stored in different second storage parts 81, and the objects to be manipulated to be introduced can be switched. Specifically, it is possible to adopt, for example, a configuration in which one of the second storage parts 81 stores an object to be manipulated for machine learning, for example, a bead or the like, another one of the second storage parts 81 stores an object to be manipulated as a research target, for example, a cell or the like, the second storage part 81 is selected according to each scene of the machine learning and the research, and a desired object to be manipulated is introduced into the manipulation region 60.

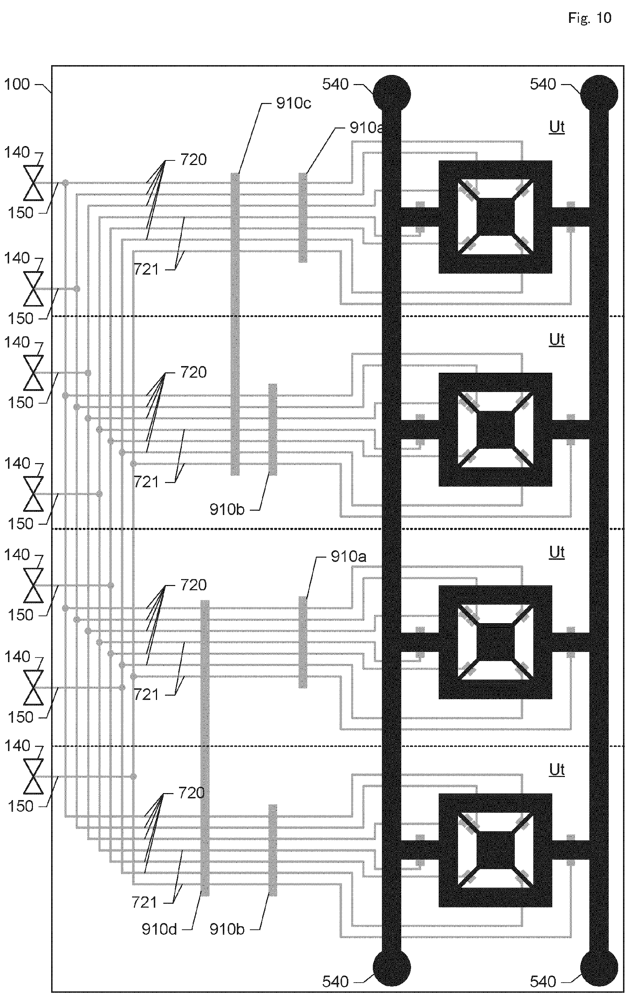

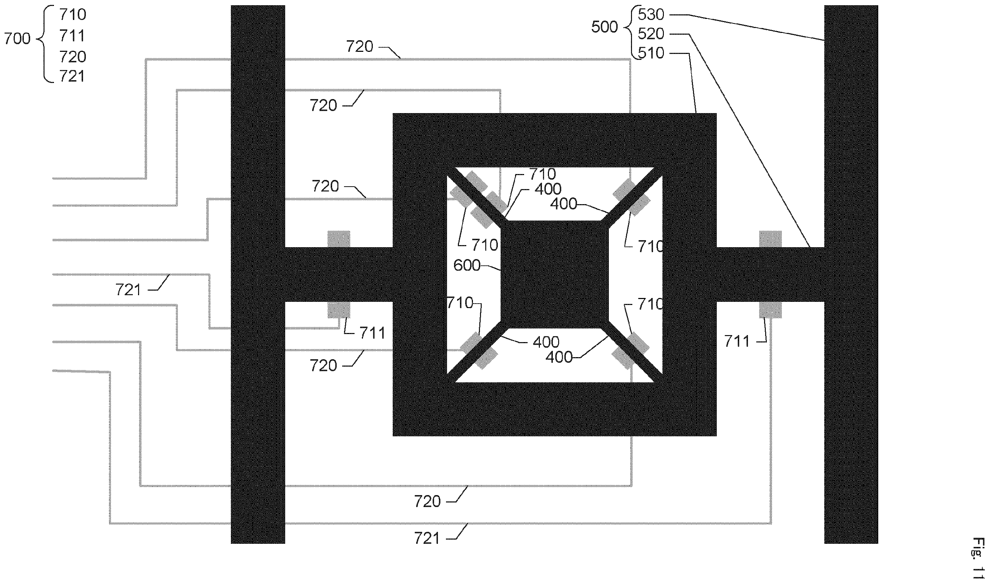

Furthermore, the manipulation region is not limited to one region, and the disposition of the object to be manipulated may be manipulatable using a plurality of manipulation regions. Figs. 10 and 11 are diagrams for explaining a manipulation system including a plurality of manipulation regions. Fig. 10 is a diagram illustrating a substrate 100 for realizing the manipulation system, and Fig. 11 is an enlarged view of a portion around a manipulation region provided on the substrate 100.

The manipulation system is realized by the substrate 100 including a plurality of units Ut. In Fig. 10, the boundaries of the units Ut are indicated by broken lines. A plurality of electromagnetic valves 140 are connected to the substrate 100. The electromagnetic valves 140 are connected to a signal conversion interface (not illustrated). The signal conversion interface can be realized by a circuit having a function similar to that of the signal conversion interface illustrated in Fig. 1. In addition, similarly to the embodiment illustrated in Fig. 1, a computer (not illustrated) is connected to the signal conversion interface, and a microscope (not illustrated) is connected to the computer. The computer and the microscope also only need to have functions equivalent to those of the computer 11 and the microscope 12.

That is, a machine learning model trained with machine-learning in advance for the manipulation regions and the like provided on the substrate 100 is recorded in a storage medium (not illustrated) of the computer. In addition, the computer acquires an image of the manipulation regions to be described later of the respective units Ut with the microscope, outputs a signal to the signal conversion interface on the basis of the image, and controls the electromagnetic valves 140 to manipulate disposition of an object to be manipulated in the manipulation regions.

A compressor (not illustrated) and a pressure supply pipe 150 are connected to each electromagnetic valve 140, and connection between the compressor and the pressure supply pipe 150 is switched by opening and closing the electromagnetic valve 140. Therefore, the electromagnetic valve 140 can switch an internal pressure of the pressure supply pipe 150 between a high state and a low state.

The pressure supply pipe 150 is connected to an air pressure adjustment unit 700 (described later) formed on the substrate 100. Therefore, the electromagnetic valve 140 can switch an internal pressure of the air pressure adjustment unit 700 to which the pressure supply pipe 150 is connected between a high state and a low state.

Each of the plurality of units Ut provided on the substrate 100 is provided with a liquid-containing structure containing liquid and a structure containing gas. Specifically, each of the units Ut includes a first channel 400, a second channel 500, a manipulation region 600, and the air pressure adjustment unit 700. Also in the present embodiment, the liquid is water, and the gas is air. Although the object to be manipulated is introduced into the first channel 400 or the manipulation region 600, a configuration for introducing the object to be manipulated is omitted in Figs. 10 and 11.

The first channel 400, the second channel 500, and the manipulation region 600 are channels and a region containing the liquid. The manipulation region 600 is a region containing the liquid, in which the disposition of the object to be manipulated is manipulated, and has an internal space in which the object to be manipulated is moved planarly. In the present embodiment, the manipulation region 600 and the like are formed on the largest surface of the substrate 100, and the internal space of the manipulation region 600 has a shape that enables planar movement of the object to be manipulated in a direction parallel to the largest surface of the substrate 100. That is, the manipulation region 600 has an internal space extending in the direction parallel to the largest surface of the substrate 100 at a fixed depth (for example, a depth of 30 µm or less) in the largest surface. Furthermore, the internal space of the manipulation region 600 has a quadrangular shape as viewed from a direction perpendicular to the largest surface of the substrate 100.

The first channel 400 is a linear hollow space, one end of which is connected to each vertex of the quadrangle formed by the manipulation region 600. In the present embodiment, the first channel 400 is a channel extending in a direction parallel to a diagonal line of the quadrangle formed by the manipulation region 600. The other end of the first channel 400 is connected to a channel 510 that is a part of the second channel 500. In the present embodiment, the depth of the first channel 400 is the same as the depth of the manipulation region 600, and the width of the first channel 400 is smaller than the width of the second channel 500.

The second channel 500 includes the channel 510, a channel 520, a channel 530, and a storage part 540. The channel 510 is a square frame-shaped channel formed around the manipulation region 600. In the channel 510, the first channel 400 is connected to the inner peripheral side of each vertex portion of the square. The channel 530 is a channel extending in a direction in which the units Ut are arranged, and is a channel formed at two positions with the channel 510 therebetween.

The channel 510 is a series of channels connecting four of the first channels 400, and the channel 520 is a channel connecting the channel 510 and the channel 530. The depths of the channel 510, the channel 520, and the channel 530 are the same as that of the first channels 400. The widths of the channel 510, the channel 520, and the channel 530 are larger than the width of the first channels 400.

The storage part 540 is a circular portion and is connected to an end of the channel 530. The storage part 540 has a larger depth than that of the first channels 400 (for example, 3 mm to 5 mm). In addition, the diameter of the circle formed by the storage part 540 is larger than the width of the channel 530.

The air pressure adjustment unit 700 is a structure including a passage containing the gas. In the present embodiment, the air pressure adjustment unit 700 includes switching parts 710 and 711 (see Fig. 11) and gas passages 720 and 721. Of course, a portion such as the connection part 73 illustrated in Fig. 1B described above may be provided at a connection portion between the pressure supply pipe 150 and the gas passage 720.

The switching part 710 includes a mechanism capable of switching the first channel 400 between a closed state and a non-closed state. In the present embodiment, one or more of the switching parts 710 are provided in each of the four first channels 400. The total number of switching parts 710 is preferably the number of first channels 400 + 1. In the example illustrated in Figs. 10 and 11, two switching parts 710 are provided in the first channel 400 present at the upper left of the manipulation region 600, and one switching part 710 is provided in each of the other first channels 400. Therefore, the total number of switching parts 710 is 5. The switching parts 710 and the first channels 400 also have structures as illustrated in Figs. 2C to 2E described above, and internal pressures of the first channels 400 can be adjusted by the switching parts 710.

The gas passage 720 is a gas passage extending from each of the switching parts 710. In the respective units Ut, the gas passages 720 extend from the switching parts 710 toward the electromagnetic valves 140 and further extend in the direction in which the units Ut are arranged, whereby the gas passages 720 of the respective units Ut are connected to each other. In the present embodiment, the gas passages 720 extending from the switching parts 710 having the same relative positional relationship with respect to the manipulation regions 600 among the switching parts 710 present in the respective units Ut are connected to each other. For example, in Figs. 10 and 11, the gas passages 720 extending from the switching parts 710 present at the upper right of the manipulation regions 600 are connected to each other.

In the substrate 100, the pressure supply pipe 150 extending from one electromagnetic valve 140 is connected to one gas passage 720. Therefore, when the internal pressure of the pressure supply pipe 150 becomes high or low under the control of the electromagnetic valve 140, an internal pressure of the gas passage 720 also becomes high or low. As a result, a state in which the liquid cannot flow and a state in which the liquid can flow in the first channel 400 can be switched.

In this manner, in the present embodiment, the air pressure adjustment unit 700 constitutes a structure that applies, to the liquid, a pressure for moving the liquid in the first channels. In addition, the electromagnetic valves 140 and the pressure supply pipes 150 constitute a pressure supply unit that supplies a pressure to the structures included in the plurality of units Ut.

In the present embodiment, the switching part 711 is also formed in the channel 520, and the gas passage 721 is connected to the switching part 711. The structures of the switching part 711 and the gas passage 721 are similar to the structures of the switching part 710 and the gas passage 720. Therefore, when the internal pressure of the pressure supply pipe 150 becomes high or low under the control of the electromagnetic valve 140, an internal pressure of the gas passage 721 also becomes high or low. As a result, a state in which the liquid cannot flow and a state in which the liquid can flow in the channel 520 can be switched.

In the state in which the liquid cannot flow in the channel 520, the flow of the liquid between the channel 510 and the channel 530 is blocked, so that the pressure of the channel 510 included in each unit Ut can be prevented from being affected by a pressure fluctuation due to manipulation or the like in another unit Ut. In the state in which the liquid can flow in the channel 520, the liquid can flow between the channel 510 and the channel 530, so that the pressures in the respective channels 510 included in the plurality of units Ut can be made uniform.

In the present embodiment, the substrate 100 further includes supply control units 910a to 910d that switch each of the plurality of units Ut between a state in which pressure supply to the air pressure adjustment unit 700 is enabled and a state in which the pressure supply is disabled (see Fig. 10). That is, the supply control units 910a to 910d can switch between a state in which the gas can flow and a state in which the gas cannot flow in the intersecting gas passages 720 and 721.

The supply control units 910a to 910d can be realized by various configurations, and for example, a pressure may be applied to the diaphragms or the like as illustrated in Figs. 2C to 2E by the electromagnetic valves and the pressure supply pipes to block or open the gas passages 720 and 721. Alternatively, the gas passages 720 and 721 may be directly blocked or opened by the electromagnetic valves.

Each of the supply control units 910a to 910d simultaneously switches whether to supply a pressure in the plurality of intersecting gas passages 720 and 721. In the example illustrated in Fig. 10, each of the supply control units 910a to 910d simultaneously switches whether to supply a pressure in 14 intersecting gas passages. For example, the supply control unit 910a simultaneously switches whether to supply a pressure in five gas passages 720 and two gas passages 721 in the unit Ut illustrated at the uppermost stage in Fig. 10, and five gas passages 720 and two gas passages 721 in the unit Ut illustrated at the third stage from the top. On the other hand, the supply control unit 910c simultaneously switches whether to supply a pressure in five gas passages 720 and two gas passages 721 in the unit Ut illustrated at the uppermost stage in Fig. 10, and five gas passages 720 and two gas passages 721 in the unit Ut illustrated at the second stage from the top.

The supply control units 910 are connected to the signal conversion interface (not illustrated), and the computer controls the operations of the supply control units 910a to 910d via the signal conversion interface. That is, the computer can select any unit Ut and enable or disable the adjustment of the air pressure by the electromagnetic valves 140 by switching a combination of blocking or opening of the gas passages by the supply control units 910a to 910d.

For example, assume a case in which the pressure supply is disabled by the supply control units 910a and 910c and the pressure supply is enabled by the supply control units 910b and 910d. In this case, the uppermost unit Ut and the third unit Ut from the top illustrated in Fig. 10 are disabled by the supply control unit 910a disabling the pressure supply. In addition, the second unit Ut from the top is disabled by the supply control unit 910c disabling the pressure supply. On the other hand, the lowermost unit Ut illustrated in Fig. 10 is enabled by the supply control units 910b and 910d enabling the pressure supply.

The pressure supply by the electromagnetic valves 140 can be performed in the enabled unit Ut, and switching by the switching parts 710 and 711 is performed through the gas passages 720 and 721. That is, the pressure on the liquid in the first channels 400 is adjusted and the channel 520 is opened and closed. With the above configuration, in the present embodiment, it is possible to select any unit Ut and manipulate the disposition of the object to be manipulated in the manipulation region 600 of the selected unit Ut under the control of the computer.

Furthermore, the method of connecting the second channel to all the first channels in the configuration in which the position of the object to be manipulated in the manipulation region is controlled by the movement of the liquid from the plurality of first channels can also be realized as a method invention. In addition, the system and the method as described above can be assumed to be realized as a single device or realized by a plurality of devices, and include various modes. For example, it is possible to provide a microscope or the like provided with the above-described means, or an attachment to be mounted on an existing microscope. Furthermore, the invention is also established as a recording medium of the program executed by the control unit. Of course, the recording medium of the software may be a magnetic recording medium or a semiconductor memory, and can be considered exactly the same in any recording medium to be developed in the future.

### REFERENCE SIGNS LIST

- 10: Manipulation system
- 11: Computer
- 12: Microscope
- 13: Signal conversion interface
- 14: Electromagnetic valve
- 15: Pressure supply pipe
- 16: Micro-fluid chip
- 20: Control unit
- 21: Manipulation program
- 21a: Imaging module
- 21b: Position control module
- 22: Machine learning program
- 22a: Machine learning module
- 30: Storage medium
- 30b: Machine learning model
- 30c: Simulation model
- 40: First channel
- 50: Second channel
- 51: Storage part
- 52: Coupling part
- 60: Manipulation region
- 70: Air pressure adjustment unit
- 71: Switching part
- 71a: Diaphragm
- 71b: Space
- 72: Gas passage
- 73: Connection part
- 80: Third channel
- 81: Second storage part
- 82: Second switching part
- 83: Second gas passage
- 84: Second connection part

## Claims

1. A manipulation system for manipulating disposition of an object to be manipulated at a predetermined position in a manipulation region containing liquid, the manipulation system comprising:
the manipulation region;
a first channel that is a plurality of channels connected to the manipulation region and containing the liquid;
a liquid control unit configured to move the liquid in the first channel; and
a second channel that is a series of channels containing the liquid, the second channel being connected to each of the plurality of first channels.

2. The manipulation system according to claim 1, wherein the manipulation region has an internal space in which the object to be manipulated is moved along a plane, and the internal space has a polygonal shape as viewed from a direction perpendicular to the plane.

3. The manipulation system according to claim 2, wherein the first channel is connected to a vertex of the polygonal shape.

4. The manipulation system according to claim 1 or 2, wherein
the first channel is thinner than the second channel.

5. The manipulation system according to claim 1, wherein the liquid control unit includes
a switching part configured to switch the first channel between a closed state and a non-closed state to move the liquid,
one or more of the switching parts are provided in each of the first channels, and
a total number of the switching parts to be provided is equal to or more than the number of first channels + 1.

6. The manipulation system according to claim 1, wherein the liquid control unit includes
a switching part configured to switch the first channel between a closed state and a non-closed state to move the liquid, and
two of the switching parts are provided in the single first channel.

7. The manipulation system according to claim 5 or 6, wherein
the liquid control unit operates the switching part on a basis of output obtained by inputting a position of the object to be manipulated, a state of the switching part, and a target position of the object to be manipulated to a machine learning model having been trained with machine-learning such that a position of the object to be manipulated, a state of the switching part, and a target position of the object to be manipulated are input, and an operation in the switching part necessary for bringing the object to be manipulated close to a target position is output.

8. The manipulation system according to claim 1 or 2, wherein
the second channel includes
a coupling part and a storage part for the liquid larger than the coupling part,
the storage part is connected to the first channel, and
the coupling part is formed to couple a plurality of the storage parts between the storage parts.

9. A fluid chip comprising:
a manipulation region containing liquid, in which disposition of an object to be manipulated is manipulated;
a first channel that is a plurality of channels connected to the manipulation region and containing the liquid;
a structure configured to apply, to the liquid, a pressure for moving the liquid in the first channel; and
a second channel that is a series of channels containing the liquid, the second channel being connected to each of the plurality of first channels.

10. A manipulation system comprising
a plurality of units each including:
a manipulation region containing liquid, in which disposition of an object to be manipulated is manipulated;
a first channel that is a plurality of channels connected to the manipulation region and containing the liquid;
a structure configured to apply, to the liquid, a pressure for moving the liquid in the first channel; and
a second channel that is a series of channels containing the liquid, the second channel being connected to each of the plurality of first channels,
the second channels included in the plurality of units being connected to each other,
the manipulation system further comprising:
a pressure supply unit configured to supply the pressure to the structures included in the plurality of units, and
a supply control unit configured to switch each of the plurality of units between a state in which supply of the pressure from the pressure supply unit to the structure is enabled and a state in which the supply is disabled.
